# EUROPÄISCHE PATENTSCHRIFT

(11) **EP 3 078 345 B1**
(45) Veröffentlichungstag und Bekanntmachung des Hinweises auf die Patenterteilung: **27.05.2020**
(21) Anmeldenummer: 15161217.3
(22) Anmeldetag: 27.03.2015
(51) Int. Cl.: A61C 1/08, A61C 1/00, A61B 90/00

(54) **MEDIZINISCHES ODER DENTALES INSTRUMENTENTEIL**
MEDICAL OR DENTAL INSTRUMENT PORTION
PARTIE D'INSTRUMENT MÉDICAL OU DENTAIRE

(43) Veröffentlichungstag der Anmeldung: 12.10.2016
(60) Teilanmeldung: 19210529.4
(73) Patentinhaber: W & H Dentalwerk Bürmoos GmbH, 5111 Bürmoos (AT)
(72) Erfinder: Pruckner, Christian, 1190 Wien (AT); Silberer, Bernhard, 5152 Michaelbeuern (AT); Eibl, Johann, 5230 Mattighofen (AT)
(74) Vertreter: Benda, Ralf

(56) Entgegenhaltungen:
- EP-A1- 2 184 028
- EP-A1- 2 514 386
- EP-A1- 2 727 552
- US-A1- 2003 165 794

## Beschreibung

Die vorliegende Erfindung betrifft ein medizinisches oder dentales Instrumententeil mit einer mit elektrischer Energie betreibbaren Speichervorrichtung zur Speicherung von Identifikationsdaten und / oder Betriebsdaten und / oder Pflegedaten des Instrumententeils.

Ein medizinisches oder dentales Instrumententeil mit einer mit elektrischer Energie betreibbaren Speichervorrichtung ist zum Beispiel aus der Patentanmeldung US 2003/0165794 A1 bekannt. Die Speichervorrichtung ist Teil einer Identifikationssignal-Ausgabevorrichtung zum aktiven Ausgeben von Identifikationssignalen, um das Instrumententeil zu identifizieren, so dass ein Schaltkreis einer Steuer-, Regel- oder Versorgungseinheit automatisch das Instrumententeil erkennt und entsprechend versorgt oder antreibt. Die Energieversorgung und Kommunikation zwischen der Speichervorrichtung und dem Antriebsschaltkreis erfolgt drahtgebunden über elektrische Leitungen, welche die Speichervorrichtung und die Steuer-, Regel- oder Versorgungseinheit elektrisch verbinden. An dem Instrumententeil ist auch ein Verbindungsanschluss für eine Lampe vorgesehen.

Der vorliegenden Erfindung liegt die Aufgabe zugrunde den Aufbau eines medizinischen oder dentalen Instrumententeils in Hinblick auf die drahtgebundene Energieversorgung und Kommunikationsverbindung/Datenübertragung zwischen der Speichervorrichtung und der Steuer-, Regel- oder Versorgungseinheit zu verbessern. Insbesondere soll der Aufbau des Instrumententeils einfach sein und die zur Energieversorgung und Kommunikationsverbindung zwischen der Speichervorrichtung und der Steuer-, Regel- oder Versorgungseinheit benötigten Bauteile wenig Platz einnehmen und eine zuverlässige Energieversorgung und Datenübertragung gewährleisten.

Diese Aufgabe wird gemäß der vorliegenden Erfindung durch ein medizinisches oder dentales Instrumententeil mit den Merkmalen des Anspruchs 1, durch eine medizinische oder dentale Behandlungsvorrichtung mit den Merkmalen des Anspruchs 10 und durch ein Verfahren zum Betrieb eines medizinischen oder dentalen Instrumententeils oder einer medizinischen oder dentalen Behandlungsvorrichtung mit den Merkmalen des Anspruchs 14 gelöst. Besonders vorteilhafte Ausführungsformen sind in den Unteransprüchen angeführt.

Das medizinische oder dentale Instrumententeil umfasst: eine Kupplungsvorrichtung zur Verbindung des Instrumententeils mit einer Steuer-, Regel- oder Versorgungseinheit, eine mit elektrischer Energie betreibbare Speichervorrichtung zur Speicherung von Identifikationsdaten und / oder Betriebsdaten und / oder Pflegedaten des Instrumententeils und nicht mehr als zwei, exakt zwei elektrische Leitungen, die für die Versorgung einer an dem Instrumententeil vorgesehenen oder mit dem Instrumententeil verbundenen oder verbindbaren Beleuchtungsvorrichtung mit elektrischer Energie zum Betrieb der Beleuchtungsvorrichtung und für die Versorgung der Speichervorrichtung mit elektrischer Energie zum Betrieb der Speichervorrichtung vorgesehen ist.

Durch das Vorsehen der nicht mehr als zwei, exakt zwei elektrischen Leitungen, die für die Versorgung der Beleuchtungsvorrichtung mit elektrischer Energie zum Betrieb der Beleuchtungsvorrichtung und für die Versorgung der Speichervorrichtung mit elektrischer Energie zum Betrieb der Speichervorrichtung vorgesehen sind, wird der Aufbau des Instrumententeils erheblich vereinfacht. So wird zum Beispiel die Anzahl an elektrischen Leitungen in dem Instrumententeil reduziert. Insbesondere wird der Aufbau der Kupplungsvorrichtung zur Verbindung des Instrumententeils mit einer Steuer-, Regel- oder Versorgungseinheit außerordentlich vereinfacht, da die Anzahl an elektrischen Kontakten an der Schnittfläche der Kupplungsvorrichtung reduziert ist.

Die nicht mehr als zwei, exakt zwei elektrischen Leitungen, die für die Versorgung der an dem Instrumententeil vorgesehenen oder mit dem Instrumententeil verbindbaren Beleuchtungsvorrichtung mit elektrischer Energie zum Betrieb der Beleuchtungsvorrichtung und für die Versorgung der Speichervorrichtung mit elektrischer Energie zum Betrieb der Speichervorrichtung vorgesehen sind, sind als gemeinsame elektrische Leitungen für die Beleuchtungsvorrichtung und die Speichervorrichtung ausgebildet. Die Beleuchtungsvorrichtung und die Speichervorrichtung sind somit elektrisch mit den nicht mehr als zwei, exakt zwei gemeinsamen elektrischen Leitungen verbunden. Besonders bevorzugt ist eine Verzweigung vorgesehen, von welcher sich eine erste elektrische Zweigleitung zu der Speichervorrichtung und eine zweite elektrische Zweigleitung zu der Beleuchtungsvorrichtung erstreckt.

Die nicht mehr als zwei, exakt zweigemeinsamen elektrischen Leitungen sind vorzugsweise als Drahtleitung und / oder als auf einer Leiterplatte gedruckte elektrische Leitung ausgebildet.

Unter dem Begriff ,medizinisches oder dentales Instrumententeil' wird im Folgenden insbesondere verstanden: ein mit einer Hand haltbares medizinisches oder dentales Element, ein Handgriffelement, ein gerades Handstück, ein gewinkeltes oder gebogenes Handstück oder Winkelstück, ein Adapter, ein Kupplungselement, ein Antriebselement, insbesondere ein Luftmotor oder ein E-Motor, oder ein Versorgungsschlauch oder ein Teil der im Vorstehenden genannten Elemente.

Vorzugsweise umfasst das Instrumententeil ein medizinisches oder dentales Handstück oder zumindest einen Teil davon, das aufweist: ein in Drehung versetzbares Antriebselement zum Antrieb eines mit dem Handstück lösbar verbindbaren Werkzeugs, eine Kupplungsvorrichtung zur Verbindung des Handstücks mit einer Steuer-, Regel- oder Versorgungseinheit, eine mit elektrischer Energie betreibbare Speichervorrichtung zur Speicherung von Identifikationsdaten und / oder Betriebsdaten und / oder Pflegedaten des Handstücks, eine mit elektrischer Energie betreibbare Beleuchtungsvorrichtung zur Abgabe von Strahlung auf eine Behandlungsstelle und nicht mehr als zwei, exakt zweigemeinsame elektrische Leitungen, die für die Versorgung der Beleuchtungsvorrichtung mit elektrischer Energie zum Betrieb der Beleuchtungsvorrichtung und der Speichervorrichtung mit elektrischer Energie zum Betrieb der Speichervorrichtung vorgesehen sind.

Die Kupplungsvorrichtung zur Verbindung des Instrumententeils mit einer Steuer-, Regel- oder Versorgungseinheit ist insbesondere als lösbare Kupplungsvorrichtung ausgebildet, so dass das Instrumententeil von der Steuer-, Regel- oder Versorgungseinheit trennbar ist. Die Kupplungsvorrichtung ist zum Beispiel als Steckkupplung oder als Drehkupplung ausgebildet, so dass das Instrumententeil relativ zu der Steuer-, Regel- oder Versorgungseinheit drehbar ist, wenn das Instrumententeil mit dem Steuer-, Regel- oder Versorgungseinheit über die Kupplungsvorrichtung verbunden ist.

Die Kupplungsvorrichtung umfasst insbesondere eine Stirnfläche, an welcher zumindest ein elektrischer Kontakt vorgesehen ist, der mit den nicht mehr als zwei, exakt zweigemeinsamen elektrischen Leitungen verbunden ist. Vorzugsweise sind zwei elektrische Kontakte an der Stirnfläche der Kupplungsvorrichtung angeordnet, wobei jeweils ein elektrischer Kontakt mit einer der nicht mehr als zwei, exakt zwei gemeinsamen elektrischen Leitungen verbunden ist. Wenn zumindest eine separate optische Leitung zum Übertragen von Identifikationsdaten und / oder Betriebsdaten und / oder Pflegedaten vorhanden ist, dann ist vorzugsweise in entsprechender Weise ein optischer Kontakt vorgesehen, der mit dieser zumindest einen optischen Leitung für die Datenübertragung verbunden ist.

Vorzugsweise ist der zumindest eine elektrische Kontakt oder sind die elektrischen Kontakte lösbar mit der Steuer-, Regel- oder Versorgungseinheit verbunden. Der zumindest eine elektrische Kontakt ist zum Beispiel als stiftförmiger elektrischer Kontakt, als Federkontakt, als Schleifkontakt oder als ringförmiger, ein Bauteil der Kupplungsvorrichtung umgebender elektrischer Kontakt ausgebildet.

Die Speichervorrichtung umfasst entweder einen nur lesbaren Speicher (ROM) oder vorzugsweise einen beschreibbaren und lesbaren Speicher. Die Speichervorrichtung ist insbesondere als digitale Speichervorrichtung zur Speicherung und / oder zum Auslesen und / oder zum Verarbeiten digitaler Daten ausgebildet.

Vorzugsweise sind in der Speichervorrichtung des Instrumententeils insbesondere Identifikationsdaten und / oder Betriebsdaten und / oder Pflegedaten gespeichert oder speicherbar, die jenem Instrumententeil zugeordnet sind, in dem die Speichervorrichtung angeordnet ist. Alternativ ist es auch denkbar, das in der Speichervorrichtung ausschließlich oder zusätzlich Identifikationsdaten und / oder Betriebsdaten und / oder Pflegedaten von einem anderen als jenem Instrumententeil, in dem die Speichervorrichtung angeordnet ist, speicherbar oder gespeichert sind.

Die Speichervorrichtung umfasst vorzugsweise ein Speicherelement und einen Mikrocontroller oder Mikrocomputer, die wahlweise als ein Bauteil oder als zwei getrennte, elektrisch miteinander verbundene Bauteile ausgebildet sind. Wenn das Speicherelement und der Mikrocontroller getrennt voneinander ausgebildet sind, ist es möglich sie an unterschiedlichen Stellen des Instrumententeils zu positionieren, wodurch die Anordnung der beiden Bauteile in einem Instrumententeil mit geringem inneren Raumvolumen erleichtert wird.

Die Speichervorrichtung mit dem Mikrocontroller ist vorzugsweise zum aktiven Ausgeben eines digitalen Signals oder von digitalen Daten ausgebildet. Die Speichervorrichtung mit dem Mikrocontroller umfasst insbesondere auch Software, die es ermöglicht Daten zu empfangen, abzusenden, zu verarbeiten, zu speichern und / oder eigenständige Steuerschritte durchzuführen.

Die Versorgung der Beleuchtungsvorrichtung und der Speichervorrichtung mit elektrischer Energie sowie die (digitale) Datenübertragung über die nicht mehr als zwei, exakt zwei gemeinsame elektrischen Leitungen ist/sind zum Beispiel durch Aufmodulieren und / oder durch digitales Multiplexen und / oder durch zeitlich versetztes Senden oder Übertragen von digitalen Daten und / oder elektrischer Energie realisierbar, insbesondere mit Hilfe des Mikrocontrollers der Speichervorrichtung.

Die an dem Instrumententeil vorgesehene oder mit dem Instrumententeil verbindbare Beleuchtungsvorrichtung umfasst vorzugsweise eine oder mehrere Lichtquellen, zum Beispiel optische Halbleiterelemente, insbesondere Leuchtdioden (LEDs). Vorzugsweise umfasst die Beleuchtungsvorrichtung mehrere Lichtquellen, wobei zumindest eine erste Lichtquelle (LED) eine erste Wellenlänge oder einen ersten Wellenlängenbereich und zumindest eine zweite Lichtquelle (LED) eine zweite Wellenlänge oder einen zweiten Wellenlängenbereich emittiert, die von der ersten Wellenlänge oder dem ersten Wellenlängenbereich unterschiedlich ist. Bevorzugt sind die erste Lichtquelle und die zweite Lichtquelle in unterschiedlichen elektrischen Stromrichtungen angeordnet. Besonders bevorzugt ist an dem Instrumententeil, insbesondere mit dem Mikrocontroller verbunden oder als Teil des Mikrocontrollers, oder an einem mit dem Instrumententeil verbundenen oder verbindbaren Element, zum Beispiel der Steuer-, Regel- oder Versorgungseinheit, eine Schaltvorrichtung zum wahlweisen Betrieb der ersten Lichtquelle oder der zweiten Lichtquelle und / oder zur wahlweisen Emission von elektromagnetischer Strahlung durch die erste Lichtquelle oder durch die zweite Lichtquelle vorgesehen. Besonders bevorzugt steuert die Schaltvorrichtung den wahlweisen Betrieb der ersten Lichtquelle oder der zweiten Lichtquelle über die zumindest eine (gemeinsame) elektrische Leitung. Besonders bevorzugt ist die Schaltvorrichtung als elektrische Schaltvorrichtung ausgebildet, zum Beispiel als H-Brücke.

Wenn das Instrumententeil als medizinisches oder dentales Handstück ausgebildet ist, so wie dies im Vorstehenden beschrieben ist, so ist die Beleuchtungsvorrichtung vorzugsweise anschließend an einen oder an einem Kopfteil des Handstücks, in dem die Werkzeughalterung für das lösbar verbindbare Werkzeug gelagert ist, angeordnet. Insbesondere umfasst die Beleuchtungsvorrichtung mehrere optische Halbleiterelemente, die ringförmig um eine Werkzeugaufnahmeöffnung des Kopfteils positioniert sind.

Alternativ umfasst das Instrumententeil mit der Speichervorrichtung, insbesondere mit dem Mikrocontroller, keine Beleuchtungsvorrichtung, sondern nur zumindest eine (gemeinsame) elektrische Leitung, die neben der Versorgung der Speichervorrichtung mit elektrischer Energie auch zur Versorgung der Beleuchtungsvorrichtung mit elektrischer Energie vorgesehen ist. Die Beleuchtungsvorrichtung ist in einem anderen Element angeordnet, das mit dem Instrumententeil mit der Speichervorrichtung derart verbindbar oder verbunden ist, dass die Beleuchtungsvorrichtung über die nicht mehr als zwei, exakt zwei gemeinsameelektrischen Leitungen mit elektrischer Energie versorgbar ist.

Erfindungsgemäß sind die nicht mehr als zwei, exakt zwei gemeinsamen elektrischen Leitungen für die Versorgung der Beleuchtungsvorrichtung mit elektrischer Energie zum Betrieb der Beleuchtungsvorrichtung und der Speichervorrichtung mit elektrischer Energie zum Betrieb der Speichervorrichtung und zum Übertragen von Identifikationsdaten und / oder Betriebsdaten und / oder Pflegedaten zwischen der Speichervorrichtung und der mit dem Instrumententeil verbindbaren Steuer-, Regel- oder Versorgungseinheit vorgesehen. Die Versorgung der Beleuchtungsvorrichtung und der Speichervorrichtung mit elektrischer Energie sowie die Datenübertragung erfolgen damit ausschließlich über diese nicht mehr als zwei, exakt zwei gemeinsamen elektrischen Leitungen. Diese Ausführungsform ist somit in Bezug auf den Aufbau des medizinischen oder dentalen Instrumententeils, der Anzahl der elektrischen Kontakte an der Kupplungsvorrichtung und des Platzverbrauchs die einfachste oder optimale Ausführungsform.

Vorzugsweise sind die Beleuchtungsvorrichtung und die Speichervorrichtung mit dem Mikrocontroller, insbesondere einschließlich einer der Speichervorrichtung zugeordneten Spannungsvorrichtung, elektrisch parallel angeordnet.

Vorzugsweise ist ein, insbesondere der Speichervorrichtung zugeordnetes, elektrisches Schaltelement vorgesehen, das ausgebildet ist, Änderungen eines elektrischen Stromparameters, insbesondere der elektrischen Spannung, zur Übertragung von Identifikationsdaten und / oder Betriebsdaten und / oder Pflegedaten zwischen der Speichervorrichtung und der mit dem Instrumententeil verbindbaren Steuer-, Regel- oder Versorgungseinheit zu bewirken. Vorzugsweise ist das elektrische Schaltelement zum Unterstützen des Auslesens oder zum Auslesen von Daten aus der Speichervorrichtung vorgesehen, insbesondere zur Übertragen von Daten von der Speichervorrichtung zu der Steuer-, Regel- oder Versorgungseinheit. Vorzugsweise öffnet und schließt das elektrische Schaltelement einen Schaltkreis zwischen der Speichervorrichtung und der Steuer-, Regel-oder Versorgungseinheit, wodurch ein elektrischer Stromparameter, insbesondere die elektrische Spannung, veränderbar ist, zum Beispiel ein elektrischer Stromparameter der elektrischen Energie zur Versorgung der Beleuchtungsvorrichtung und der Speichervorrichtung. Vorzugsweise ist das elektrische Schaltelement mit den nicht mehr als zwei, exakt zwei gemeinsamen elektrischen Leitungen elektrisch verbunden. Vorzugsweise sind die nicht mehr als zwei, exakt zwei gemeinsamen elektrischen Leitungen Teil dieses Schaltkreises. Wie im Weiteren noch im Detail beschrieben wird, ist die Steuer-, Regel- oder Versorgungseinheit ausgebildet, die Änderung des elektrischen Stromparameters zu empfangen, wobei die Änderung des Stromparameters bzw. die Reaktion der Steuer-, Regel- oder Versorgungseinheit die übertragenen Identifikationsdaten und / oder Betriebsdaten und / oder Pflegedaten, insbesondere digital, definieren.

Vorzugsweise ist das elektrische Schaltelement ausgebildet, zum Auslesen von Daten aus der Speichervorrichtung den im folgenden Absatz beschriebenen Shunt-Widerstand kurzzuschließen.

Vorzugsweise ist ein, insbesondere der Speichervorrichtung zugeordneter, Shunt-Widerstand vorgesehen, der ausgebildet ist, Änderungen der elektrischen Stromstärke zur Übertragung von Identifikationsdaten und / oder Betriebsdaten und / oder Pflegedaten zwischen der Speichervorrichtung und der mit dem Instrumententeil verbindbaren Steuer-, Regel- oder Versorgungseinheit aufzubereiten. Vorzugsweise ist der Shunt-Widerstand zum Abspeichern von Daten in der Speichervorrichtung vorgesehen oder er unterstützt dieses, insbesondere zur Übertragen von Daten von der Steuer-, Regel- oder Versorgungseinheit zu der Speichervorrichtung. Vorzugsweise wandelt der Shunt-Widerstand Werte der elektrischen Stromstärke der Steuer-, Regel- oder Versorgungseinheit, welche Identifikationsdaten und / oder Betriebsdaten und / oder Pflegedaten (digital) definieren, in von der Speichervorrichtung verarbeitbare Spannungswerte, welche Identifikationsdaten und / oder Betriebsdaten und / oder Pflegedaten (digital) definieren. Vorzugsweise ist der Shunt-Widerstand mit den nicht mehr als zwei, exakt zwei gemeinsamen elektrischen Leitungen elektrisch verbunden.

Bevorzugt ist der Shunt-Widerstand alternativ oder zusätzlich zur Stromüberwachung für die Beleuchtungsvorrichtung, insbesondere für das zumindest eine optische Halbleiterelement, ausgebildet. Insbesondere verhindert der Shunt-Widerstand eine Versorgung der Beleuchtungsvorrichtung mit einer zu hohen elektrischen Stromstärke.

Vorzugsweise ist eine der Speichervorrichtung, insbesondere dem Mikrocontroller, zugeordnete Vorrichtung zur Spannungsaufbereitung vorgesehen, die ausgebildet ist, der Speichervorrichtung, insbesondere dem Mikrocontroller, eine konstante elektrische Spannung zuzuführen. Insbesondere bildet die Spannungsaufbereitung eine konstante Spannungsquelle für die Speichervorrichtung, insbesondere den Mikrocontroller. Vorzugsweise ist die Spannungsaufbereitung mit einer Energiequelle, insbesondere mit einer elektrischen Konstantstromquelle, in der Steuer-, Regel- oder Versorgungseinheit elektrisch verbunden, insbesondere über die nicht mehr als zwei, exakt zwei gemeinsamen elektrischen Leitungen. Vorzugsweise versorgt die Energiequelle, insbesondere die Konstantstromquelle, in der Steuer-, Regel- oder Versorgungseinheit die Spannungsaufbereitung mit elektrischer Energie. Vorzugsweise ist die Spannungsaufbereitung derart ausgebildet, dass die Speichervorrichtung, insbesondere der Mikrocontroller, mit einer geringeren elektrischen Spannung versorgt wird als die Beleuchtungsvorrichtung. Vorzugsweise ist die Spannungsaufbereitung in Serie mit der Speichervorrichtung angeordnet.

Vorzugsweise ist eine der Beleuchtungsvorrichtung zugeordnete Vorrichtung zur Spannungsüberwachung vorgesehen, die ausgebildet ist, die der Beleuchtungsvorrichtung zugeführte elektrische Spannung zu überwachen. Die Spannungsüberwachung umfasst insbesondere einen Spannungsteiler, der die Eingangsspannung am Instrumententeil überwacht. Der Spannungsteiler weist zum Beispiel zwei elektrische Widerstände auf, die mit dem Mikrocontroller der Speichervorrichtung verbunden sind.

Gemäß einem Ausführungsbeispiel umfasst das Instrumententeil zumindest einen Sensor, der ausgebildet ist, einen Betriebszustand des Instrumententeils zu detektieren und ein, vorzugsweise elektrisches, Sensorsignal zu erzeugen, wobei der zumindest eine Sensor mit den nicht mehr als zwei, exakt zwei gemeinsamen elektrischen Leitungen elektrisch verbunden ist, so dass das Sensorsignal des Sensors und / oder elektrische Energie zum Betreiben des Sensors über die nicht mehr als zwei, exakt zwei gemeinsamen elektrischen Leitungen übertragbar ist/sind.

Der Sensor weist zum Beispiel einen Temperatursensor zum Messen der Temperatur des Instrumententeils oder eines Abschnitts davon oder zumindest eines in dem Instrumententeil angeordneten Bauteils, insbesondere eines durch ein Antriebselement in Bewegung versetzbaren Bauteils, zum Beispiel eines Lagers, auf. Der Temperatursensor umfasst insbesondere einen elektrisch betreibbaren Temperatursensor, zum Beispiel einen Temperatursensor mit einem Material, dessen elektrischer Widerstand sich mit der Temperatur verändert (z.B. NTC-Temperatursensor), oder einen Infrarot-Temperatursensor.

Der Sensor weist alternativ zum Beispiel einen Drehzahlsensor zum Messen der Drehzahl eines Antriebselements des Instrumententeils oder eines damit verbindbaren Werkzeugs auf. Der Drehzahlsensor ist zum Beispiel als induktiver, kapazitiver oder optischer Drehzahlsensor ausgebildet.

Selbstverständlich kann der Sensor auch ausgebildet sein, andere Parameter zu messen, und zum Beispiel einen Sensor zum Messen einer Leistung, von Kraft oder Druck, eines Drehmoments, einer Lichtintensität oder Wellenlänge, etc. aufweisen.

Vorzugsweise wird das Sensorsignal über die nicht mehr als zwei, exakt zwei gemeinsamen elektrischen Leitungen an die Steuer-, Regel- oder Versorgungseinheit, insbesondere an einen darin angeordneten Mikrocontroller, geleitet. Der zumindest eine Sensor ist somit insbesondere derart über die nicht mehr als zwei, exakt zwei gemeinsamen elektrischen Leitungen mit der Steuer-, Regel- oder Versorgungseinheit verbunden, dass die Steuer-, Regel- oder Versorgungseinheit ein Sensorsignal empfängt und verarbeitet. Insbesondere ist die Steuer-, Regel- oder Versorgungseinheit ausgebildet, das Instrumententeil auf Basis eines von dem zumindest einem Sensor erhaltenen Sensorsignal zu steuern, zu regeln oder, zum Beispiel mit einem Betriebsmittel, zu versorgen.

Gemäß einem Ausführungsbeispiel ist eine medizinische oder dentale Behandlungsvorrichtung vorgesehen, die umfasst: ein medizinisches oder dentales Instrumententeil wie im Vorstehenden oder nachfolgend beschrieben, eine elektrische Energiequelle, insbesondere eine Konstantstromquelle, die ausgebildet ist, die Beleuchtungsvorrichtung und die Speichervorrichtung des Instrumententeils mit elektrischer Energie zu versorgen, und eine Steuer-, Regel- oder Versorgungseinheit, die zum Austausch von Identifikationsdaten und / oder Betriebsdaten und / oder Pflegedaten des Instrumententeils mit der Speichervorrichtung des Instrumententeils ausgebildet ist. Vorzugsweise sind das Instrumententeil und die Steuer-, Regel- oder Versorgungseinheit über die Kupplungsvorrichtung lösbar miteinander verbunden.

Vorzugsweise ist in der Steuer-, Regel- oder Versorgungseinheit ein weiterer Mikrocontroller vorgesehen, der mit dem Mikrocontroller des Instrumententeils verbunden ist, wobei die nicht mehr als zwei, exakt zwei gemeinsamen elektrischen Leitungen einen Teil dieser Verbindung bilden. Vorzugsweise ist auch die Energiequelle in der Steuer-, Regel- oder Versorgungseinheit angeordnet und insbesondere über die nicht mehr als zwei, exakt zwei gemeinsamen elektrischen Leitungen mit der Beleuchtungsvorrichtung und der Speichervorrichtung elektrisch verbunden.

Wie im Vorstehenden bereits beschrieben versorgt die Energiequelle, insbesondere die Konstantstromquelle, die Beleuchtungsvorrichtung mit einer konstanten Stromstärke. Die Speichervorrichtung, insbesondere der Mikrocontroller, die eine konstante elektrische Spannung benötigt, wird von der vorzugsweise als Konstantstromquelle ausgebildeten Energiequelle über eine der Speichervorrichtung zugeordnete Vorrichtung zur Spannungsaufbereitung mit elektrischer Energie versorgt. Die Vorrichtung zur Spannungsaufbereitung ist ausgebildet, der Speichervorrichtung eine konstante elektrische Spannung zuzuführen.

Vorzugsweise ist/sind die Steuer-, Regel- oder Versorgungseinheit, insbesondere deren Mikrocontroller, ausgebildet, zum Auslesen der in der Speichervorrichtung gespeicherten Identifikationsdaten und / oder Betriebsdaten und / oder Pflegedaten, insbesondere durch das Schaltelement bewirkte, Änderungen der elektrischen Last oder der elektrischen Spannung zu detektieren oder zu erkennen.

Wie im Vorstehenden bereits beschrieben öffnet und schließt das elektrische Schaltelement vorzugsweise einen Schaltkreis zwischen der Speichervorrichtung und der Steuer-, Regel- oder Versorgungseinheit und schließt insbesondere den der Speichervorrichtung zugeordneten Shunt-Widerstand kurz, wodurch ein elektrischer Stromparameter, insbesondere die elektrische Spannung, welcher die Identifikationsdaten und / oder Betriebsdaten und / oder Pflegedaten (digital) definiert, veränderbar ist. Die Steuer-, Regel- oder Versorgungseinheit, insbesondere deren Mikrocontroller, sind ausgebildet, die von dem Schaltelement bewirkten Änderungen oder Schwankungen zu erkennen oder zu detektieren. Die Änderung des Stromparameters bzw. das Ausgleichen dieser Schwankungen des elektrischen Stromparameters werden von dem Mikrocontroller der Steuer-, Regel- oder Versorgungseinheit erfasst und daraus die von der Speichervorrichtung übertragenen Identifikationsdaten und / oder Betriebsdaten und / oder Pflegedaten erkannt.

Vorzugsweise ist/sind die elektrische Energiequelle, insbesondere die Konstantstromquelle, und / oder die Steuer-, Regel- oder Versorgungseinheit ausgebildet, zum Übertragen von Daten an die Speichervorrichtung die elektrische Stromstärke, welche dem, insbesondere der Speichervorrichtung zugeordneten, Shunt-Widerstand und der Speichervorrichtung zuführbar ist, zu variieren. Der Mikrocontroller der Steuer-, Regel- oder Versorgungseinheit ist insbesondere ausgebildet, die Änderung der elektrischen Stromstärke zu bewirken oder zu steuern. Die Änderung der Stromstärke definiert, insbesondere digital, die Identifikationsdaten und / oder Betriebsdaten und / oder Pflegedaten. Wie im Vorstehenden beschrieben, wandelt vorzugsweise ein Shunt-Widerstand die Werte der elektrischen Stromstärke der Steuer-, Regel- oder Versorgungseinheit, welche Identifikationsdaten und / oder Betriebsdaten und / oder Pflegedaten definieren, in elektrische Spannungswerte um, die von dem Mikrocontroller des Instrumententeils empfangbar und verarbeitbar sind, so dass der Mikrocontroller Daten auf dem Speicherelement der Speichervorrichtung abspeichern kann.

Vorzugsweise ist die Steuer-, Regel- oder Versorgungseinheit ausgebildet, das Instrumententeil auf Basis von in der Speichervorrichtung gespeicherten und aus der Speichervorrichtung ausgelesenen Identifikationsdaten und / oder Betriebsdaten und / oder Pflegedaten und / oder auf Basis des von dem zumindest einem Sensor erhaltenen Sensorsignals zu betreiben. Zum Beispiel ist die Steuer-, Regel- oder Versorgungseinheit ausgebildet, auf Basis dieser Daten einem Instrumententeil die auf dieses Instrumententeil abgestimmte Antriebsenergie und / oder zumindest ein bestimmtes Medium und / oder eine bestimmte Medienmenge zuzuleiten.

Vorzugsweise ist die Steuer-, Regel- oder Versorgungseinheit ausgebildet, auf das Instrumententeil zum Beispiel Daten bezüglich der Dauer des Betriebs des Instrumententeils, der Art seiner Verwendung und / oder der Art und / oder Dauer seiner Reinigung oder Pflege zu übertragen.

Erfindungsgemäß ist ein Verfahren zum Betrieb eines medizinischen oder dentalen Instrumententeils oder einer medizinischen oder dentalen Behandlungsvorrichtung vorgesehen, bei dem nicht mehr als zwei, exakt zwei gemeinsame elektrische Leitungen eine an dem Instrumententeil vorgesehene oder mit dem Instrumententeil verbundene oder verbindbare Beleuchtungsvorrichtung zum Betrieb der Beleuchtungsvorrichtung und eine Speichervorrichtung zum Betrieb der Speichervorrichtung mit elektrischer Energie versorgen.

Erfindungsgemäß versorgen die nicht mehr als zwei, exakt zwei elektrische Leitungen die Beleuchtungsvorrichtung zum Betrieb der Beleuchtungsvorrichtung und die Speichervorrichtung zum Betrieb der Speichervorrichtung mit elektrische Energie und übertragen die Identifikationsdaten und / oder Betriebsdaten und / oder Pflegedaten zwischen der Speichervorrichtung und der mit dem Instrumententeil verbindbaren Steuer-, Regel- oder Versorgungseinheit.

Vorzugsweise übertragen die nicht mehr als zwei, exakt zwei gemeinsamen elektrischen Leitungen zusätzlich ein Sensorsignal des Sensors im Instrumententeil und / oder elektrische Energie zum Betreiben des Sensors, so wie dies im Vorstehenden beschrieben ist.

Vorzugsweise detektiert/detektieren oder erkennt/erkennen die Steuer-, Regel- oder Versorgungseinheit, insbesondere deren Mikrocontroller, zum Auslesen der in der Speichervorrichtung gespeicherten Identifikationsdaten und / oder Betriebsdaten und / oder Pflegedaten durch das Schaltelement bewirkte Änderungen eines elektrischen Stromparameters, insbesondere der elektrischen Spannung. Die Änderung des elektrischen Stromparameters bzw. das Ausgleichen dieser Schwankungen erfasst der Mikrocontroller der Steuer-, Regel- oder Versorgungseinheit und erkennt daraus die von der Speichervorrichtung übertragenen Identifikationsdaten und / oder Betriebsdaten und / oder Pflegedaten, so wie dies im Vorstehenden beschrieben ist.

Vorzugsweise variiert/variieren die elektrische Energiequelle, insbesondere die Konstantstromquelle, und / oder die Steuer-, Regel- oder Versorgungseinheit zum Übertragen von Daten an die Speichervorrichtung die elektrische Stromstärke, welche dem Shunt-Widerstand und der Speichervorrichtung zugeführt wird. Der Shunt-Widerstand wandelt vorzugsweise die Werte der elektrischen Stromstärke der Steuer-, Regel- oder Versorgungseinheit, welche Identifikationsdaten und / oder Betriebsdaten und / oder Pflegedaten definieren, in elektrische Spannungswerte um, welche Identifikationsdaten und / oder Betriebsdaten und / oder Pflegedaten (digital) definieren. Diese elektrischen Spannungswerte werden von dem Mikrocontroller des Instrumententeils empfangen oder verarbeitet und auf das Speicherelement gespeichert, so wie dies im Vorstehenden beschrieben ist.

Vorzugsweise betreibt die Steuer-, Regel- oder Versorgungseinheit das Instrumententeil auf Basis von in der Speichervorrichtung gespeicherten und aus der Speichervorrichtung ausgelesenen Identifikationsdaten und / oder Betriebsdaten und / oder Pflegedaten und / oder auf Basis des von dem zumindest einem Sensor erhaltenen Sensorsignals, sowie dies im Vorstehenden beschrieben ist.

Die Erfindung wird nachfolgend anhand bevorzugter Ausführungsbeispiele und Bezug nehmend auf die beigefügten Zeichnungen erläutert.
Figur 1 zeigt eine medizinische oder dentale Behandlungsvorrichtung, umfassend: ein medizinisches oder dentales Instrumententeil mit einer Beleuchtungsvorrichtung, einer Speichervorrichtung und nicht mehr als zwei, exakt zwei gemeinsamen elektrischen Leitungen für die Versorgung der Beleuchtungsvorrichtung und der Speichervorrichtung mit elektrischer Energie, eine Antriebsvorrichtung, einen Versorgungsschlauch und eine Steuer-, Regel- oder Versorgungseinheit mit einer elektrischen Energiequelle zur Versorgung der Beleuchtungsvorrichtung und der Speichervorrichtung mit elektrischer Energie.
Figur 2 zeigt das medizinische oder dentale Instrumententeil der Figur 1.
Figur 3 zeigt eine schematische Darstellung eines ersten Ausführungsbeispiels eines elektrischen Schalt- oder Stromkreises mit nicht mehr als zwei, exakt zwei gemeinsamen elektrischen Leitungen für die Versorgung einer Beleuchtungsvorrichtung und einer Speichervorrichtung.
Figur 4 zeigt eine schematische Darstellung eines zweiten Ausführungsbeispiels eines elektrischen Schalt- oder Stromkreises, das nicht Teil der Erfindung ist, mit zumindest einer gemeinsamen elektrischen Leitung für die Versorgung einer Beleuchtungsvorrichtung und einer Speichervorrichtung.
Figur 5A-5C zeigen drei unterschiedliche Ausführungsformen einer Beleuchtungsvorrichtung und eines Sensors für einen elektrischen Schalt- oder Stromkreis der Figuren 3 oder 4.

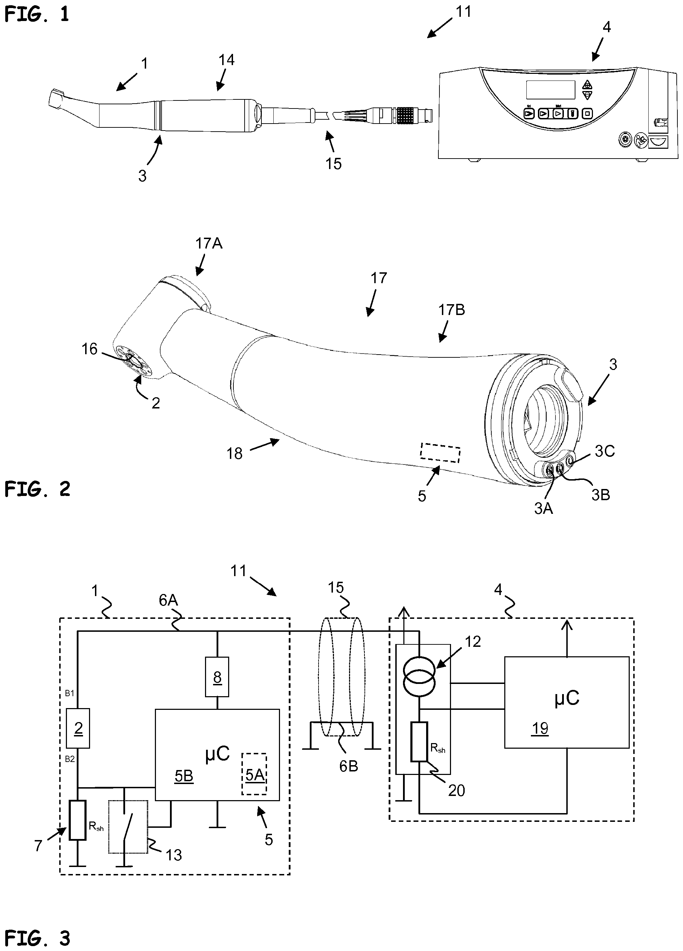

Die Figur 1 zeigt eine medizinische oder dentale Behandlungsvorrichtung 11, die ein medizinisches oder dentales Instrumententeil 1, eine Antriebseinheit 14, eine Steuer-, Regel- oder Versorgungseinheit 4 und einen das Instrumententeil 1 mit der Steuer-, Regel- oder Versorgungseinheit 4 verbindenden Versorgungsschlauch 15 umfasst. Das Instrumententeil 1 ist über eine Kupplungsvorrichtung 3 lösbar mit der Steuer-, Regel- oder Versorgungseinheit 4 verbunden.

Das in der Figur 2 vergrößert dargestellte Instrumententeil 1 ist durch ein gebogenes Handstück oder Winkelstück 17 gebildet. Das Handstück 17 umfasst einen Handstückkopf 17A, in dem eine Werkzeughalterung zur, insbesondere lösbaren, Befestigung eines Werkzeugs angeordnet ist, sowie einen Griffteil 17B zum Halten der Handstücks 17 mit einer Hand. Am freien oder proximalen Ende des Griffteils 17B ist die Kupplungsvorrichtung 3 bzw. zumindest ein Teil davon angeordnet.

Die Antriebseinheit 14 umfasst zum Beispiel einen E-Motor oder einen Luftmotor, die als separates, von dem Handstück 17 lösbares Bauteil (wie in Figur 1 dargestellt) oder in das Handstück 17 integriert ausgebildet sind. Die Antriebseinheit 14 kann jedoch auch ein in das Handstück 17, insbesondere im Handstückkopf 17A, angeordnetes, mit einem Treibgas angetriebenes Laufrad umfassen. Das Handstück 17 kann somit motorbetrieben sein und zumindest eine Antriebswelle zum Übertragen einer Antriebsbewegung auf das Werkzeug aufweisen oder treibgasbetrieben mit einem Laufrad ausgebildet sein. In beiden Fällen umfasst das Handstück 17 zumindest ein in Drehung versetzbares Antriebselement zum Antrieb eines mit dem Handstück verbindbaren Werkzeugs.

Das Handstück 17 umfasst des Weiteren eine Beleuchtungsvorrichtung 2, die vorzugsweise am Handstückkopf 17A oder daran anschließend angeordnet ist. Die in der Figur 2 dargestellte Beleuchtungsvorrichtung 2 umfasst insbesondere mehrere optische Halbleiterelemente (LEDs), die ringförmig um eine Werkzeugaufnahmeöffnung 16 des Handstückkopfs 17A angeordnet sind.

Bevorzugt sind des Weiteren um die Werkzeugaufnahmeöffnung 16, vorzugsweise alternierend mit den optischen Halbleiterelementen, Öffnungen zur Abgabe eines Mediums, zum Beispiel von Wasser und / oder Luft, angeordnet. Die Öffnungen zur Abgabe eines Mediums sind über zumindest eine Medienleitung in dem Handstück 17 mit der Steuer-, Regel- oder Versorgungseinheit 4 zum Zuführen zumindest eines Mediums verbunden.

In dem Instrumententeil 1 oder Handstück 17 ist des Weiteren eine mit elektrischer Energie betreibbare Speichervorrichtung 5 zur Speicherung von Identifikationsdaten und / oder Betriebsdaten und / oder Pflegedaten des Instrumententeils 1 oder Handstücks 17 angeordnet. Die Speichervorrichtung 5 kann im Handstückkopf 17A oder im Griffteil 17B angeordnet sein oder es kann jeweils ein Teil der Speichervorrichtung 5 im Handstückkopf 17A und im Griffteil 17B vorgesehen sein. Innerhalb des Griffteils 17B ist die Speichervorrichtung 5 oder ein Teil davon zum Beispiel an dessen proximalen oder freien Ende, insbesondere in oder an der Kupplungsvorrichtung 3, in einem unmittelbar an den Handstückkopf 17A anschließenden Abschnitt des Griffteils 17B oder in einer Biegung 18 des Griffteils 17B angeordnet.

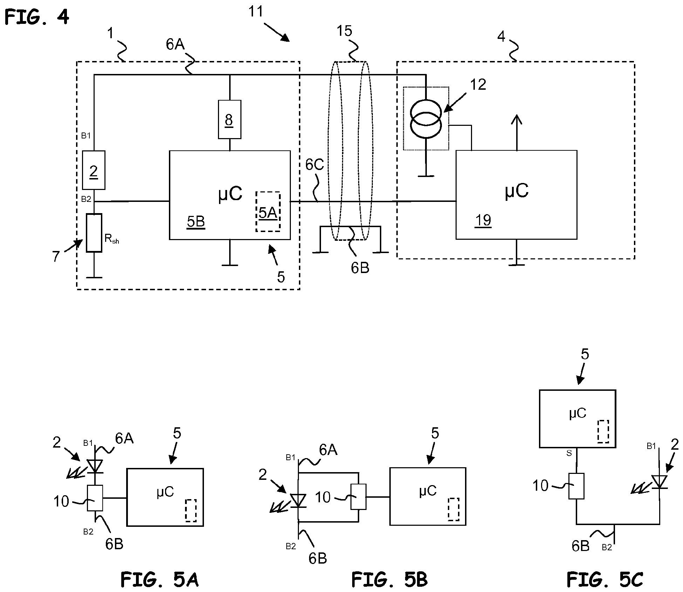

Die Speichervorrichtung 5 umfasst insbesondere ein Speicherelement 5A und einen Mikrocontroller 5B, insbesondere zum Betreiben des Speicherelements 5A (siehe Figuren 3 und 4).

Wie insbesondere aus den Figuren 3 und 4 zu erkennen ist, umfasst das Instrumententeil 1 oder das Handstück 17 des Weiteren gemäß der Figur 3 nicht mehr als zwei, exakt zwei gemeinsame elektrische Leitungen 6A, 6B und gemäß der Figur 4, die nicht Teil der Erfindung ist, zumindest eine (gemeinsame) elektrische Leitung 6A, 6B, die für die Versorgung der an dem Handstück 17 vorgesehenen Beleuchtungsvorrichtung 2 mit elektrischer Energie zum Betrieb der Beleuchtungsvorrichtung 2 und für die Versorgung der Speichervorrichtung 5 mit elektrischer Energie zum Betrieb der Speichervorrichtung 5 vorgesehen sind/ ist. Die zumindest eine (gemeinsame) elektrische Leitung 6A, 6B oder die nicht mehr als zwei, exakt zwei gemeinsamen elektrischen Leitungen 6A, 6B verbindet/ verbinden die Beleuchtungsvorrichtung 2 und die Speichervorrichtung 5 mit einer in der Steuer-, Regel- oder Versorgungseinheit 4 angeordneten elektrischen Energiequelle 12, insbesondere eine Konstantstromquelle, die ausgebildet ist, die Beleuchtungsvorrichtung 2 und die Speichervorrichtung 5 mit elektrischer Energie zu versorgen. Wie aus den Figuren 3 und 4 auch zu erkennen ist, erstreckt sich die zumindest eine (gemeinsame) elektrische Leitung 6A, 6B oder erstrecken sich die nicht mehr als zwei, exakt zwei gemeinsamen elektrischen Leitungen 6A, 6B durch den Versorgungsschlauch 15 bis zu oder in die Steuer-, Regel- oder Versorgungseinheit 4.

Die zumindest eine (gemeinsame) elektrische Leitung 6A, 6B oder die nicht mehr als zwei, exakt zwei gemeinsamen elektrischen Leitungen 6A, 6B verbindet/ verbinden somit elektrisch die Beleuchtungsvorrichtung 2 und die Speichervorrichtung 5 mit der Steuer-, Regel- oder Versorgungseinheit 4, insbesondere deren elektrischer Energiequelle 12 und Mikrocontroller 19, wodurch ein elektrischer Schalt-, Steuer-, Regel- oder Versorgungskreis gebildet ist. Vorzugsweise umfasst der Schalt-, Steuer-, Regel- oder Versorgungskreis auch noch zumindest einen Sensor 10, wie im Folgenden noch im Detail beschrieben ist.

Die elektrische Energiequelle 12 in Form einer Konstantstromquelle versorgt das Instrumententeil 1 oder das Handstück 17 mit elektrischer Energie mit konstanter elektrischer Stromstärke. Dies bildet für die zumindest einen optischen Halbleiter umfassende Beleuchtungsvorrichtung 2 eine optimale Energieversorgung. Die Speichervorrichtung 5, insbesondere der Mikrocontroller 5B, benötigt jedoch eine elektrische Versorgung mit einer konstanten elektrischen Spannung. Dazu ist eine der Speichervorrichtung 5, insbesondere dem Mikrocontroller 5B, zugeordnete Vorrichtung 8 zur Spannungsaufbereitung vorgesehen, die ausgebildet ist, die von der elektrischen Energiequelle 12 (über die elektrische Leitung 6A, 6B) empfangene elektrischer Energie mit konstanter elektrischer Stromstärke in elektrische Energie mit konstanter elektrischer Spannung zu wandeln und der Speichervorrichtung 5 zuzuführen. Die Vorrichtung 8 zur Spannungsaufbereitung ist insbesondere in dem Instrumententeil 1 oder dem Handstück 17 angeordnet.

Gemäß der Figur 3 sind nicht mehr als zwei, exakt zwei gemeinsame elektrische Leitungen 6A, 6B für die Versorgung der Beleuchtungsvorrichtung 2 und der Speichervorrichtung 5 mit elektrischer Energie vorgesehen, so wie dies im Vorstehenden beschrieben ist. Zusätzlich sind die nicht mehr als zwei, exakt zwei elektrischen Leitungen 6A, 6B zum Übertragen von Identifikationsdaten und / oder Betriebsdaten und / oder Pflegedaten zwischen der Speichervorrichtung 5 und der mit dem Instrumententeil 1 oder Handstück 17 verbindbaren Steuer-, Regel- oder Versorgungseinheit 4 vorgesehen. In anderen Worten sind somit für die Versorgung der Beleuchtungsvorrichtung 2 und der Speichervorrichtung 5 mit elektrischer Energie und für die Datenübertragung nicht mehr als die zwei, exakt diese zwei (gemeinsamen) elektrischen Leitungen 6A, 6B vorgesehen. Entsprechend ist die Steuer-, Regel- oder Versorgungseinheit 4 zum Austausch von Identifikationsdaten und / oder Betriebsdaten und / oder Pflegedaten des Instrumententeils 1 mit der Speichervorrichtung 5 ausgebildet.

Zur Übertragung von Identifikationsdaten und / oder Betriebsdaten und / oder Pflegedaten von der Speichervorrichtung 5 und zu der Steuer-, Regel- oder Versorgungseinheit 4, d.h. zum Auslesen von Daten aus der Speichervorrichtung 5, ist ein der Speichervorrichtung 5 zugeordnetes elektrisches Schaltelement 13 vorgesehen. Wie im Vorstehenden bereits beschrieben ist das Schaltelement 13 ausgebildet, Änderungen eines elektrischen Stromparameters zu bewirken, insbesondere der elektrischen Spannung oder der elektrischen Last, insbesondere durch das Kurzschließen eines Shunt-Widerstands 7. Die Änderungen des elektrischen Stromparameters definieren (digital) die übertragenen Daten. Die Steuer-, Regel- oder Versorgungseinheit 4, insbesondere deren Mikrocontroller 19, ist ausgebildet, die Änderungen des elektrischen Stromparameters zu detektieren oder auszugleichen und daraus die (digital) übertragenen Identifikationsdaten und / oder Betriebsdaten und / oder Pflegedaten zu erkennen oder auszulesen.

Im Instrumententeil 1 oder Handstück 17 ist des Weiteren einen Shunt-Widerstand 7 vorgesehen, der ausgebildet ist, Änderungen der elektrischen Stromstärke zur Übertragung von Identifikationsdaten und / oder Betriebsdaten und / oder Pflegedaten zwischen der Speichervorrichtung 5 und der mit dem Instrumententeil 1 verbindbaren Steuer-, Regel- oder Versorgungseinheit 4 aufzubereiten. Wie im Vorstehenden bereits beschrieben ist der Shunt-Widerstand 7 zum Abspeichern von Daten in der Speichervorrichtung 5 vorgesehen, insbesondere zur Übertragen von Daten von der Steuer-, Regel- oder Versorgungseinheit 4 zu der Speichervorrichtung 5.

Ein weiterer Shunt-Widerstand 20 ist der Steuer-, Regel- oder Versorgungseinheit 4, insbesondere dem Mikrocontroller 19 der Steuer-, Regel- oder Versorgungseinheit 4, zugeordnet.

Gemäß der Figur 4, die nicht Teil der Erfindung ist, sind zwei (gemeinsame) elektrische Leitungen 6A, 6B für die Versorgung der Beleuchtungsvorrichtung 2 und der Speichervorrichtung 5 mit elektrischer Energie vorgesehen, so wie dies im Vorstehenden beschrieben ist. Zum Übertragen von Identifikationsdaten und / oder Betriebsdaten und / oder Pflegedaten zwischen der Speichervorrichtung 5 und der mit dem Instrumententeil 1 oder Handstück 17 verbindbaren Steuer-, Regel- oder Versorgungseinheit 4 ist eine separate Leitung 6C oder Datenleitung vorgesehen. In anderen Worten sind somit für die Versorgung der Beleuchtungsvorrichtung 2 und der Speichervorrichtung 5 mit elektrischer Energie und für die Datenübertragung drei Leitungen vorgesehen, nämlich die zwei (gemeinsamen) elektrischen Leitungen 6A, 6B für die Versorgung mit elektrischer Energie und die separate Leitung 6C für die Übertragung der Daten, so wie dies im Vorstehenden beschrieben ist.

Die separate Datenleitung 6C kann als elektrische Leitung zum Übertragen elektrischer (Daten-)Signale oder als optische Leitung, zum Beispiel als Glasfaser, zum Übertragen optischer (Daten-)Signale ausgebildet sein. Die separate Leitung 6C erstreckt sich durch das Instrumententeil 1 oder Handstück 17 und den Versorgungsschlauch 15 bis zu oder in die Steuer-, Regel- oder Versorgungseinheit 4.

An der Kupplungsvorrichtung 3 des Instrumententeils 1 oder Handstücks 17, insbesondere an der Stirnfläche der Kupplungsvorrichtung 3, sind elektrische Kontakte 3A, 3B vorgesehen, die mit der zumindest einen gemeinsamen elektrischen Leitung 6A, 6B oder den nicht mehr als zwei, exakt zwei gemeinsamen elektrischen Leitungen 6A, 6B verbunden sind (siehe Figur 2). Diese lösbaren elektrischen Kontakte 3A, 3B verbinden die Beleuchtungsvorrichtung 2 und die Speichervorrichtung 5 elektrisch mit der Steuer-, Regel- oder Versorgungseinheit 4, insbesondere mit deren Energiequelle 12 und Mikrocontroller 19. Wenn gemäß der Figur 3 nur zwei, d.h. nicht mehr als zwei, exakt zwei gemeinsame elektrische Leitungen 6A, 6B zur Übertragung der elektrischen Energie und von Daten vorgesehen sind, so wie dies im Vorstehenden beschrieben ist, dann sind in entsprechender Weise auch nur diese zwei elektrische Kontakte 3A, 3B an der Kupplungsvorrichtung 3 vorgesehen. Wenn gemäß der Figur 4, die nicht Teil der Erfindung ist, zumindest eine separate optische oder elektrische Leitung 6C zum Übertragen von Identifikationsdaten und / oder Betriebsdaten und / oder Pflegedaten vorhanden ist, dann ist in entsprechender Weise ein weiterer optischer oder elektrischer Kontakt 3C vorgesehen, der mit dieser zumindest einen separaten Leitung 6C für die Datenübertragung verbunden ist.

Vorzugsweise ist der zumindest eine elektrische Kontakt oder sind die elektrischen Kontakte 3A, 3B, 3C lösbar mit der Steuer-, Regel- oder Versorgungseinheit 4 verbunden. Der zumindest eine elektrische Kontakt 3A, 3B ist zum Beispiel als stiftförmiger elektrischer Kontakt, als Federkontakt, als Schleifkontakt oder als ringförmiger, ein Bauteil der Kupplungsvorrichtung umgebender elektrischer Kontakt ausgebildet.

In dem medizinischen oder dentalen Instrumententeil 1 oder Handstück 17 ist vorzugsweise zumindest einen Sensor 10 vorgesehen, der ausgebildet ist, einen Betriebszustand des Instrumententeils 1 oder Handstücks 17 zu detektieren und ein Sensorsignal zu erzeugen. Der Sensor 10 umfasst zum Beispiel einen Temperatursensor oder einen Drehzahlsensor zum Messen der Drehzahl eines Antriebselements der Instrumententeils 1 / Handstücks 17. In den Figuren 5A - 5C sind unterschiedliche Anordnungen des Sensors 10 dargestellt.

Der zumindest eine Sensor 10 ist mit der zumindest einen elektrischen Leitung 6A, 6B oder den nicht mehr als zwei, exakt zwei gemeinsamen elektrischen Leitungen 6A, 6B elektrisch verbunden, so dass das Sensorsignal des Sensors 10 und / oder elektrische Energie zum Betreiben des Sensors 10 über die elektrische(n) Leitung(en) 6A, 6B übertragbar ist/sind.

Der zumindest eine Sensor 10 ist vorzugsweise auch mit der Speichervorrichtung 5, insbesondere dem Mikrocontroller 5B, elektrisch verbunden, so dass insbesondere ein Sensorsignal des Sensors 10 an die Speichervorrichtung 5 weiterleitbar ist. Die Speichervorrichtung 5, insbesondere der Mikrocontroller 5B, sind wahlweise ausgebildet (1) das (analoge) Sensorsignal des Sensors 10 weiterzuleiten, vorzugsweise an die Steuer-, Regel- oder Versorgungseinheit 4, insbesondere an deren Mikrocontroller 19, oder (2) das analoge Sensorsignal in ein digitales Signal zu wandeln und an die Steuer-, Regel- oder Versorgungseinheit 4, insbesondere an deren Mikrocontroller 19, zu leiten oder (3) das (analoge) Sensorsignal des Sensors 10 zu empfangen und zu verarbeiten und das Instrumententeil 1 / Handstück 17 oder ein Bauteil davon auf Basis des Sensorsignals zu betreiben, zu regeln oder zu steuern. Das Weiterleiten des Sensorsignals an die Steuer-, Regel- oder Versorgungseinheit 4 gemäß (1) oder (2) erfolgt vorzugsweise über zumindest eine der Leitungen 6A, 6B, 6C.

Bei der Ausführungsform der Figur 5A ist der Sensor 10, vorzugsweise ein Sensor zum Messen der Drehzahl, elektrisch in Serie mit der Beleuchtungsvorrichtung 2 angeordnet. Bei der Ausführungsform der Figur 5B ist der Sensor 10, vorzugsweise ein Sensor zum Messen der Temperatur, elektrisch parallel mit der Beleuchtungsvorrichtung 2 angeordnet. In beiden Ausführungsformen weisen der Sensor 10 und die Beleuchtungsvorrichtung 2 eine gemeinsame elektrische Energiequelle 12 auf und sie sind mit den elektrischen Leitungen 6A, 6B elektrisch verbunden.

Bei der Ausführungsform der Figur 5C sind der Sensor 10, vorzugsweise ein Sensor zum Messen der Temperatur, und die Beleuchtungsvorrichtung 2 mit unterschiedlichen elektrischen Energiequellen zur Versorgung mit elektrischer Energie verbunden.

Die Erfindung ist nicht auf die dargestellten und beschriebenen Ausführungsbeispiele beschränkt. Des Weiteren sind alle Merkmale aller beschriebenen und dargestellten Ausführungsbeispiele miteinander kombinierbar.

## Patentansprüche

1. Medizinisches oder dentales Instrumententeil (1), umfassend: eine Kupplungsvorrichtung (3) zur Verbindung des Instrumententeils (1) mit einer Steuer-, Regel- oder Versorgungseinheit (4), und eine mit elektrischer Energie betreibbare Speichervorrichtung (5) zur Speicherung von Identifikationsdaten und / oder Betriebsdaten und / oder Pflegedaten des Instrumententeils (1), **gekennzeichnet durch**
nicht mehr als zwei, exakt zwei gemeinsame elektrische Leitungen (6A, 6B), die für die Versorgung einer an dem Instrumententeil (1) vorgesehenen oder mit dem Instrumententeil (1) verbindbaren Beleuchtungsvorrichtung (2) mit elektrischer Energie zum Betrieb der Beleuchtungsvorrichtung (2) und für die Versorgung der Speichervorrichtung (5) mit elektrischer Energie zum Betrieb der Speichervorrichtung (5) und zum Übertragen von Identifikationsdaten und / oder Betriebsdaten und / oder Pflegedaten zwischen der Speichervorrichtung (5) und der mit dem Instrumententeil (1) verbindbaren Steuer-, Regel- oder Versorgungseinheit (4) vorgesehen sind.

2. Medizinisches oder dentales Instrumententeil (1) nach Anspruch 1, **dadurch gekennzeichnet, dass**
die Speichervorrichtung (5) ein Speicherelement (5A) und einen Mikrocontroller (5B) umfasst.

3. Medizinisches oder dentales Instrumententeil (1) nach Anspruch 2, **dadurch gekennzeichnet, dass**
die Versorgung der Beleuchtungsvorrichtung (2) und der Speichervorrichtung (5) mit elektrischer Energie sowie die Übertragen von Identifikationsdaten und / oder Betriebsdaten und / oder Pflegedaten über die nicht mehr als zwei, exakt zwei gemeinsamen elektrischen Leitungen durch Aufmodulieren und / oder durch digitales Multiplexen und / oder durch zeitlich versetztes Senden oder Übertragen von digitalen Daten und / oder elektrischer Energie mit dem Microcontroller (5B) der Speichervorrichtung (5) realisiert ist.

4. Medizinisches oder dentales Instrumententeil (1) nach Anspruch 2 oder 3, **dadurch gekennzeichnet, dass**
die Speichervorrichtung (5) mit dem Mikrocontroller (5B) zum aktiven Ausgeben eines digitalen Signals oder von digitalen Daten ausgebildet ist.

5. Medizinisches oder dentales Instrumententeil (1) nach einem der vorstehenden Ansprüche, **gekennzeichnet durch**
ein der Speichervorrichtung (5) zugeordnetes elektrisches Schaltelement (13), das ausgebildet ist, Änderungen eines elektrischen Stromparameters zur Übertragung von Identifikationsdaten und / oder Betriebsdaten und / oder Pflegedaten zwischen der Speichervorrichtung (5) und der mit dem Instrumententeil (1) verbindbaren Steuer-, Regel- oder Versorgungseinheit (4) zu bewirken.

6. Medizinisches oder dentales Instrumententeil (1) nach Anspruch 5, **dadurch gekennzeichnet, dass**
das elektrische Schaltelement (13) einen Schaltkreis zwischen der Speichervorrichtung (5) und der Steuer-, Regel- oder Versorgungseinheit (4) öffnet und schließt, wodurch ein elektrischer Stromparameter, insbesondere die elektrische Spannung, veränderbar ist.

7. Medizinisches oder dentales Instrumententeil (1) nach einem der vorstehenden Ansprüche, **gekennzeichnet durch**
einen Shunt-Widerstand (7), der ausgebildet ist, Änderungen der elektrischen Stromstärke zur Übertragung von Identifikationsdaten und / oder Betriebsdaten und / oder Pflegedaten zwischen der Speichervorrichtung (5) und der mit dem Instrumententeil (1) verbindbaren Steuer-, Regel- oder Versorgungseinheit (4) aufzubereiten.

8. Medizinisches oder dentales Instrumententeil (1) nach einem der vorstehenden Ansprüche, **gekennzeichnet durch**
eine der Speichervorrichtung (5), insbesondere dem Mikrocontroller (5B), zugeordnete Vorrichtung (8) zur Spannungsaufbereitung, die ausgebildet ist, der Speichervorrichtung (5), insbesondere dem Mikrocontroller (5B), eine konstante elektrische Spannung zuzuführen und/oder eine der Beleuchtungsvorrichtung (2) zugeordnete Vorrichtung zur Spannungsüberwachung, die ausgebildet ist, die der Beleuchtungsvorrichtung (2) zugeführte elektrische Spannung zu überwachen.

9. Medizinisches oder dentales Instrumententeil (1) nach einem der vorstehenden Ansprüche, **gekennzeichnet durch**
zumindest einen Sensor (10), der ausgebildet ist, einen Betriebszustand des Instrumententeils (1) zu detektieren und ein Sensorsignal zu erzeugen, wobei der zumindest eine Sensor (10) mit den nicht mehr als zwei, exakt zwei gemeinsamen elektrischen Leitungen (6A, 6B) elektrisch verbunden ist, so dass das Sensorsignal des Sensors (10) und / oder elektrische Energie zum Betreiben des Sensors (10) über die nicht mehr als zwei, exakt zwei gemeinsamen elektrischen Leitungen (6A, 6B) übertragbar ist/sind.

10. Medizinische oder dentale Behandlungsvorrichtung (11), **gekennzeichnet durch** ein medizinisches oder dentales Instrumententeil (1) nach einem der vorstehenden Ansprüche, eine elektrische Energiequelle (12), insbesondere eine Konstantstromquelle, die ausgebildet ist, die Beleuchtungsvorrichtung (2) und die Speichervorrichtung (5) mit elektrischer Energie zu versorgen, und eine Steuer-, Regel- oder Versorgungseinheit (4), die zum Austausch von Identifikationsdaten und / oder Betriebsdaten und / oder Pflegedaten des Instrumententeils (1) mit der Speichervorrichtung (5) des Instrumententeils (1) ausgebildet ist.

11. Medizinische oder dentale Behandlungsvorrichtung (11) nach Anspruch 10, **dadurch gekennzeichnet, dass**
die Steuer-, Regel- oder Versorgungseinheit (4) ausgebildet ist, zum Auslesen der in der Speichervorrichtung (5) gespeicherten Identifikationsdaten und / oder Betriebsdaten und / oder Pflegedaten durch das Schaltelement (13) bewirkte Änderungen eines elektrischen Stromparameters, welche die Identifikationsdaten und / oder Betriebsdaten und / oder Pflegedaten definieren, zu detektieren.

12. Medizinische oder dentale Behandlungsvorrichtung (11) nach Anspruch 10 oder 11, **dadurch gekennzeichnet, dass**
die elektrische Energiequelle (12), insbesondere die Konstantstromquelle, und / oder die Steuer-, Regel- oder Versorgungseinheit (4) ausgebildet ist/sind, zum Übertragen von Daten an die Speichervorrichtung (5) die elektrische Stromstärke, welche dem Shunt-Widerstand (7) und der Speichervorrichtung (5) zuführbar ist, zu variieren.

13. Medizinische oder dentale Behandlungsvorrichtung (11) nach Anspruch 10, 11 oder 12 **dadurch gekennzeichnet, dass**
die Steuer-, Regel- oder Versorgungseinheit (4) ausgebildet ist, das Instrumententeil (1) auf Basis von in der Speichervorrichtung (5) gespeicherten und aus der Speichervorrichtung (5) ausgelesenen Identifikationsdaten und / oder Betriebsdaten und / oder Pflegedaten und / oder auf Basis des von dem zumindest einem Sensor (10) erhaltenen Sensorsignals zu betreiben.

14. Verfahren zum Betrieb eines medizinischen oder dentalen Instrumententeils (1), nach einem der Ansprüche 1 - 9 oder einer medizinischen oder dentalen Behandlungsvorrichtung (11) nach einem der Ansprüche 10 - 13, **dadurch gekennzeichnet, dass**
die nicht mehr als zwei, exakt zwei gemeinsamen elektrischen Leitungen (6A, 6B) eine an dem Instrumententeil (1) vorgesehene oder mit dem Instrumententeil (1) verbindbare Beleuchtungsvorrichtung (2) zum Betrieb der Beleuchtungsvorrichtung (2) und eine Speichervorrichtung (5) zum Betrieb der Speichervorrichtung (5) mit elektrischer Energie versorgen und Identifikationsdaten und / oder Betriebsdaten und / oder Pflegedaten zwischen der Speichervorrichtung (5) und der mit dem Instrumententeil (1) verbindbaren Steuer-, Regel- oder Versorgungseinheit (4) übertragen.

15. Verfahren zum Betrieb eines medizinischen oder dentalen Instrumententeils (1) oder einer medizinischen oder dentalen Behandlungsvorrichtung (11) nach Anspruch 14, **dadurch gekennzeichnet, dass**
ein der Speichervorrichtung (5) zugeordnetes elektrisches Schaltelement (13) Änderungen eines elektrischen Stromparameters zur Übertragung von Identifikationsdaten und / oder Betriebsdaten und / oder Pflegedaten zwischen der Speichervorrichtung (5) und der mit dem Instrumententeil (1) verbindbaren Steuer-, Regel- oder Versorgungseinheit (4) bewirkt.

## Claims

1. A medical or dental instrument part (1), comprising: a coupling device (3) for connecting the instrument part (1) to a control, regulating or supply unit (4), and a memory device (5) that can be operated with electrical power for storing identification data and/or operating and/or care data of the instrument part (1), **characterized by** not more than two, exactly two joint electrical lines (6A, 6B) which are provided for supplying electrical power to a lighting device (2) that is provided on the instrument part (1) or can be connected to the instrument part (1) for operating the lighting device (2) and for supplying electrical power to the memory device (5) for operating the memory device (5) and for transmitting identification data and/or operating data and/or care data between the memory device (5) and the control, regulating or supply unit (4) connectable to the instrument part (1).

2. The medical or dental instrument part (1) according to claim 1, **characterized in that** the memory device (5) comprises a memory element (5A) and a microcontroller (5B).

3. The medical or dental instrument part (1) according to claim 2, **characterized in that** the supply of electrical power to the lighting device (2) and the memory device (5) and the transmission of identification data and/or operating data and/or care data over the not more than two, exactly two joint electrical lines (6A, 6B) are implemented by modulation and/or digital multiplexing and/or by time offset transmission or sending of digital data and/or electrical power by the microcontroller (5B) of the memory device (5).

4. The medical or dental instrument part (1) according to claim 2 or 3, **characterized in that**
the memory device (5) with the microcontroller (5B) is configured for active output of a digital signal or digital data.

5. The medical or dental instrument part (1) according to any one of the preceding claims, **characterized by**
an electrical switching element (13) which is assigned to the memory device (5) and is configured to trigger changes in an electrical current parameter for transmitting identification data and/or operating data and/or care data between the memory device (5) and the control, regulating or supply unit (4) that can be connected to the instrument part (1).

6. The medical or dental instrument part (1) according to claim 5, **characterized in that** the electrical switching element (13) opens and closes a circuit between the memory device (5) and the control, regulating or supply unit (4) so that an electrical current parameter, in particular the electrical voltage is variable.

7. The medical or dental instrument part (1) according to any one of the preceding claims, **characterized by**
a shunt resistor (7), which is configured to process changes in the electrical amperage for transmission of identification data and/or operating data and/or care data between the memory device (5) and the control, regulating or supply unit (4) that can be connected to the instrument part (1).

8. The medical or dental instrument part (1) according to any one of the preceding claims, **characterized by**
a device (8) for voltage processing, which is assigned to the memory device (5), in particular to the microcontroller (5B) and is configured to supply a constant electrical voltage to the memory device (5), in particular to the microcontroller (5B), and/or a device for monitoring voltage, which is assigned to the lighting device (2) and is configured to monitor the electrical voltage supplied to the lighting device (2).

9. The medical or dental instrument part (1) according to any one of the preceding claims, **characterized by**
at least one sensor (10), which is configured to detect an operating state of the instrument part (1) and to generate a sensor signal, wherein the at least one sensor (10) is electrically connected to the not more than two, exactly two joint electrical lines (6A, 6B) so that the sensor signal of the sensor (10) and/or electrical power for operating the sensor (10) can be transmitted over the not more than two, exactly two joint electrical lines (6A, 6B).

10. A medical or dental treatment device (11), **characterized by**
a medical or dental instrument part (1) according to any one of the preceding claims, an electrical power source (12), in particular a constant current source, which is configured to supply electrical power to the lighting device (2) and the memory device (5), and a control, regulating or supply unit (4) which is configured for exchanging identification data and/or operating data and/or care data of the instrument part (1) with the memory device (5) of the instrument part (1).

11. The medical or dental treatment device (11) according to claim 10, **characterized in that**
the control, regulating or supply unit (4) is configured to detect changes in an electrical current parameter, which are triggered by the switching element (13) and define the identification data and/or operating data and/or care data, for the purpose of readout of the identification data and/or operating data and/or care data stored in the memory device (5).

12. The medical or dental treatment device (11) according to claim 10 or 11, **characterized in that**
the electrical power source (12), in particular the constant current source and/or the control, regulating or supply unit (4) is/are configured to vary the electrical amperage, which can be supplied to the shunt resistor (7) and the memory device (5) for transmitting data to the memory device (5).

13. The medical or dental treatment device (11) according to claim 10, 11 or 12, **characterized in that**
the control, regulating or supply unit (4) is configured to operate the instrument part (1) on the basis of identification data and/or operating data and/or care data stored in the memory device (5) and read out of the memory device (5) and/or on the basis of the sensor signal received from the at least one sensor (10).

14. A method for operating a medical or dental instrument part (1) according to any one of claims 1 - 9 or a medical or dental treatment device (11) according to any one of claims 10 - 13, **characterized in that**
the not more than two, exactly two joint electrical lines (6A, 6B) supply electrical power to the lighting device (2) that is provided on the instrument part (1) or can be connected to the instrument part (1) for operation of the lighting device (2) and to the memory device (5) for operation of the memory device (5) and transmit identification data and/or operating data and/or care data between the memory device (5) and the control, regulating or supply unit (4) that can be connected to the instrument part (1).

15. The method for operating a medical or dental instrument part (1) or a medical or dental treatment device (11) according to claim 14, **characterized in that**
an electrical switching element (13) which is assigned to the memory device (5) triggers changes in an electrical current parameter for transmitting identification data and/or operating data and/or care data between the memory device (5) and the control, regulating or supply unit (4) that can be connected to the instrument part (1).

## Revendications

1. Partie d'instrument médical ou dentaire (1) comprenant: un dispositif d'accouplement (3) pour relier la partie d'instrument (1) à une unité de commande, de régulation ou d'alimentation (4), et un dispositif de mémoire (5) pouvant fonctionner avec de l'énergie électrique pour la mise en mémoire de données d'identification et/ou de données de fonctionnement et/ou de données de soins de la partie d'instrument (1), **caractérisée par**
pas plus de deux, exactement deux lignes électriques (6A, 6B) communes, lesquelles sont prévues pour l'alimentation en énergie électrique d'un dispositif d'éclairage (2) prévu au niveau de la partie d'instrument (1) ou bien pouvant être relié à la partie d'instrument (1) pour faire fonctionner le dispositif d'éclairage (2) et pour l'alimentation du dispositif de mémoire (5) en énergie électrique pour le fonctionnement du dispositif de mémoire (5) et pour la transmission de données d'identification et/ou de données de fonctionnement et/ou de données de soins entre le dispositif de mémoire (5) et l'unité de commande, de régulation ou d'alimentation (4) pouvant être reliée à la partie d'instrument (1).

2. Partie d'instrument médical ou dentaire (1) selon la revendication 1, **caractérisée en ce que**
le dispositif de mémoire (5) comprend un élément de mémoire (5A) et un microcontrôleur (5B).

3. Partie d'instrument médical ou dentaire (1) selon la revendication 2, **caractérisée en ce que**
l'alimentation du dispositif d'éclairage (2) et du dispositif de mémoire (5) avec de l'énergie électrique ainsi que la transmission de données d'identification et/ou de données de fonctionnement et/ou de données de soins via les pas plus de deux, exactement deux lignes électriques communes sont réalisées par modulation et/ou par multiplexage numérique et/ou par envoi ou transmission décalé(e) dans le temps de données numériques et/ou d'énergie électrique avec le microcontrôleur (5B) du dispositif de mémoire (5).

4. Partie d'instrument médical ou dentaire (1) selon la revendication 2 ou 3, **caractérisée en ce que**
le dispositif de mémoire (5) avec le microcontrôleur (5B) est réalisé pour la sortie active d'un signal numérique ou de données numériques.

5. Partie d'instrument médical ou dentaire (1) selon l'une des revendications précédentes, **caractérisée par**
un élément de commutation électrique (13) associé au dispositif de mémoire (5), lequel est réalisé pour produire des modifications d'un paramètre de courant électrique pour la transmission de données d'identification et/ou de données de fonctionnement et/ou de données de soins entre le dispositif de mémoire (5) et l'unité de commande, de régulation ou d'alimentation (4) pouvant être reliée à la partie d'instrument (1).

6. Partie d'instrument médical ou dentaire (1) selon la revendication 5, **caractérisée en ce que**
l'élément de commutation électrique (13) ouvre et ferme un circuit entre le dispositif de mémoire (5) et l'unité de commande, de régulation ou d'alimentation (4) moyennant quoi un paramètre de courant électrique, en particulier la tension électrique, est modifiable.

7. Partie d'instrument médical ou dentaire (1) selon l'une des revendications précédentes, **caractérisée par**
une résistance shunt (7), laquelle est réalisée pour préparer des modifications de l'intensité de courant électrique pour la transmission de données d'identification et/ou de données de fonctionnement et/ou de données de soins entre le dispositif de mémoire (5) et l'unité de commande, de régulation ou d'alimentation (4) pouvant être reliée à la partie d'instrument (1).

8. Partie d'instrument médical ou dentaire (1) selon l'une des revendications précédentes, **caractérisée par**
un dispositif pour la préparation de la tension (8) associé au dispositif de mémoire (5), en particulier au microcontrôleur (5B), lequel est réalisé pour amener au dispositif de mémoire (5), en particulier au microcontrôleur (5B), une tension électrique constante, et/ou un dispositif de surveillance de tension associé au dispositif d'éclairage (2), lequel est réalisé pour surveiller la tension électrique amenée au dispositif d'éclairage (2).

9. Partie d'instrument médical ou dentaire (1) selon l'une des revendications précédentes, **caractérisée par**
au moins un capteur (10) qui est réalisé pour détecter un état de fonctionnement de la partie d'instrument (1) et générer un signal de détection, dans laquelle l'au moins un capteur (10) est connecté électriquement au pas plus de deux, exactement deux lignes électriques communes (6A, 6B) de sorte que le signal de détection du capteur (10) et/ou de l'énergie électrique pour faire fonctionner le capteur (10) peut/peuvent être transmis via les pas plus de deux, exactement deux lignes électriques communes (6A, 6B).

10. Dispositif de traitement médical ou dentaire (11), **caractérisé par**
une partie d'instrument médical ou dentaire (1) selon l'une des revendications précédentes, une source d'énergie électrique (12), en particulier une source de courant constant, laquelle est réalisée pour alimenter le dispositif d'éclairage (2) et le dispositif de mémoire (5) avec de l'énergie électrique, et une unité de commande, de régulation ou d'alimentation (4) réalisée pour l'échange de données d'identification et/ou de données de fonctionnement et/ou de données de soins de la partie d'instrument (1) avec le dispositif de mémoire (5) de la partie d'instrument (1).

11. Dispositif de traitement médical ou dentaire (11) selon la revendication 10, **caractérisé en ce que**
l'unité de commande, de régulation ou d'alimentation (4) est réalisée, pour la lecture des données d'identification et/ou données de fonctionnement et/ou données de soins stockées dans le dispositif de mémoire (5), pour détecter des modifications d'un paramètre de courant électrique provoquées par l'élément de commutation (13), lesquelles définissent les données d'identification et/ou données de fonctionnement et/ou données de soins.

12. Dispositif de traitement médical ou dentaire (11) selon la revendication 10 ou 11, **caractérisé en ce que**
la source d'énergie électrique (12), en particulier la source de courant constant et/ou l'unité de commande, de régulation ou d'alimentation (4) est/sont réalisées, pour la transmission de données au dispositif de mémoire (5), pour faire varier l'intensité du courant électrique qu'il est possible d'amener à la résistance shunt (7) et au dispositif de mémoire (5).

13. Dispositif de traitement médical ou dentaire (11) selon les revendications 10, 11 ou 12, **caractérisé en ce que**
l'unité de commande, de régulation ou d'alimentation (4) est réalisée pour faire fonctionner la partie d'instrument (1) sur la base de données d'identification et/ou de données de fonctionnement et/ou de données de soins stockées dans le dispositif de mémoire (5) et lues à partir du dispositif de mémoire (5) et/ou sur la base du signal de détection obtenu de l'au moins un capteur (10).

14. Procédé pour faire fonctionner une partie d'instrument médical ou dentaire (1) selon l'une des revendications 1 - 9 ou un dispositif de traitement médical ou dentaire (11) selon l'une des revendications 10 - 13, **caractérisé en ce que**
les pas plus de deux, exactement deux lignes électriques communes (6A, 6B) alimentent en énergie électrique un dispositif d'éclairage (2) prévu au niveau de la partie d'instrument (1) ou pouvant être relié à la partie d'instrument (1) pour faire fonctionner le dispositif d'éclairage (2) et un dispositif de mémoire (5) pour faire fonctionner le dispositif de mémoire (5), et assurent la transmission de données d'identification et/ou de données de fonctionnement et/ou de données de soins entre le dispositif de mémoire (5) et l'unité de commande, de régulation et d'alimentation (4) pouvant être reliée à la partie d'instrument (1).

15. Procédé pour faire fonctionner une partie d'instrument médical ou dentaire (1) ou un dispositif de traitement médical ou dentaire (11) selon la revendication 14, **caractérisé en ce que**
un élément de commutation électrique (13) associé au dispositif de mémoire (5) provoque des modifications d'un paramètre de courant électrique pour la transmission de données d'identification et/ou de données de fonctionnement et/ou de données de soins entre le dispositif de mémoire (5) et l'unité de commande, de régulation et d'alimentation (4) pouvant être reliée à la partie d'instrument (1).
